(19) Europäisches Patentamt
European Patent Office
Office européen des brevets

(11) Veröffentlichungsnummer: **0 475 236 B1**

(12) **EUROPÄISCHE PATENTSCHRIFT**

(45) Veröffentlichungstag der Patentschrift: **25.10.95**

(51) Int. Cl.[6]: **C07D 207/08**, C07D 207/26, A01N 43/36

(21) Anmeldenummer: **91114791.6**

(22) Anmeldetag: **03.09.91**

# (54) N-Acylpyrrolidin-Derivate.

(30) Priorität: **13.09.90 DE 4029054**

(43) Veröffentlichungstag der Anmeldung:
**18.03.92 Patentblatt 92/12**

(45) Bekanntmachung des Hinweises auf die Patenterteilung:
**25.10.95 Patentblatt 95/43**

(84) Benannte Vertragsstaaten:
**CH DE FR GB LI**

(56) Entgegenhaltungen:
**EP-A- 0 201 742**

**CHEMICAL ABSTRACTS, Band 52, Nr. 8, 25. April 1958, Columbus, Ohio, USA ZAKHARKIN L.I. "Action of nitric acid on 1,1,5-trichloro-1-pentene" Spalte 6175, Zusammenfassung-Nr. 6 175b & Izvest. Akad. Nauk S.S.S.R, Otdel. Khim Nauk, 1064-71 (1957)**

(73) Patentinhaber: **BAYER AG**

**D-51368 Leverkusen (DE)**

(72) Erfinder: **Negele, Michael, Dr.**
**Wolfskaul 6**
**W-5000 Köln 80 (DE)**
Erfinder: **Baasner, Bernd, Dr.**
**Wagnerstrasse 83**
**W-5060 Bergisch Gladbach 2 (DE)**
Erfinder: **Bertram, Heinz-Jürgen, Dr.**
**Schneckenbergstrasse 18**
**W-3450 Holzminden (DE)**
Erfinder: **Hartwig, Jürgen, Dr.**
**Franz-Esser-Strasse 44**
**W-5090 Leverkusen 3 (DE)**

**EP 0 475 236 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue N-Acylpyrrolidin-Derivate, Verfahren zur Herstellung der neuen N-Acylpyrrolidin-Derivate und deren Verwendung als Herbizide und Nematozide.

Es wurden neue N-Acylpyrrolidin-Derivate der allgemeinen Formel

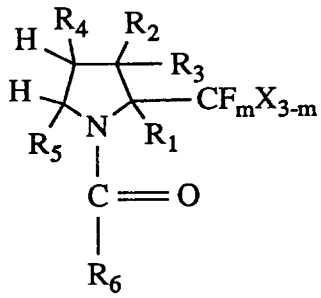

(I),

gefunden

in welcher

| | |
|---|---|
| $R_1$, $R_2$ und $R_3$ | unabhängig voneinander für Wasserstoff, Halogen, Alkyl oder Aryl stehen, |
| $R_4$ | für Wasserstoff, Halogen, Cyano, Alkyl, Aryl oder |
| | 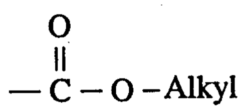 |
| | steht, |
| $R_5$ | für Wasserstoff, Alkyl oder Aryl steht, |
| $R_6$ | für Alkyl oder Aryl steht, |
| X | für Wasserstoff, Halogen oder Alkyl steht, und |
| m | für 1, 2 oder 3 steht. |

Alle Alkylreste können geradkettig oder verzweigt sein. Weiterhin können alle Alkyl- und Arylreste gegebenenfalls substituiert sein.

Weiterhin wurde gefunden, daß man die neuen N-Acylpyrrolidin-Derivate der Formel (I)

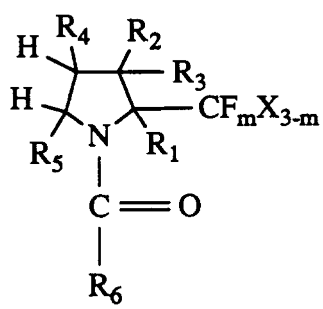

(I),

in welcher

$R_1$, $R_2$, $R_3$, $R_4$, $R_5$, $R_6$, X und m die oben angegebene Bedeutung haben, erhält, wenn man Pyrrolidin-Derivate der Formel

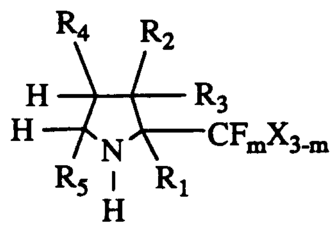 (II),

in welcher

in welcher

$R_1$, $R_2$, $R_3$, $R_3$, $R_4$, $R_5$, X und m die oben angegebene Bedeutung haben,

mit Säurehalogeniden der Formel

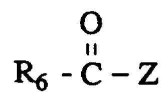 (III),

in welcher

- $R_6$ die oben angegebene Bedeutung hat, und
- Z für Halogen, insbesondere für Brom oder Chlor, steht,

gegebenenfalls in Gegenwart eines Verdünnungsmittels und gegebenenfalls in Gegenwart eines Säurebindemittels umsetzt.

Schließlich wurde gefunden, daß die neuen N-Acylpyrrolidin-Derivate der Formel (I) eine gute Wirkung als Nematizide sowie zur Bekämpfung unerwünschten Pflanzenwachstums besitzen.

Die erfindungsgemäßen neuen N-Acylpyrrolidin-Derivate sind durch die Formel (I) allgemein definiert. Bevorzugt sind Verbindungen der Formel (I), in welchen

- $R_1$, $R_2$ und $R_3$ unabhängig voneinander für Wasserstoff, Fluor, Chlor, Brom, gegebenenfalls substituiertes $C_1$-$C_6$-Alkyl oder gegebenenfalls substituiertes $C_6$-$C_{10}$-Aryl stehen,
- $R_4$ für Wasserstoff, Fluor, Chlor, Brom, Cyano, gegebenenfalls substituiertes $C_1$-$C_6$-Alkyl, gegebenenfalls substituiertes $C_6$-$C_{10}$-Aryl oder

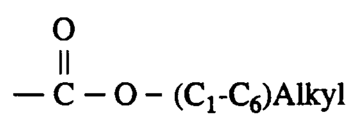

steht,

- $R_5$ für Wasserstoff, gegebenenfalls substituiertes $C_1$-$C_6$-Alkyl oder gegebenenfalls substituiertes $C_6$-$C_{10}$-Aryl steht,
- $R_6$ für gegebenenfalls substituiertes $C_1$-$C_6$-Alkyl oder gegebenenfalls substituiertes $C_6$-$C_{10}$-Aryl steht,
- X für Wasserstoff, Fluor, Chlor, Brom oder gegebenenfalls substituiertes $C_1$-$C_4$-Alkyl steht, und
- m für 1, 2 oder 3 steht,

wobei als Alkylsubstituenten in den Definitionen von $R_1$, $R_2$, $R_3$, $R_4$, $R_5$, $R_6$ und X jeweils infrage kommen Fluor, Chlor, Brom, Hydroxy, Methoxy oder Ethoxy, und als Arylsubstituenten in den Definitionen von $R_1$, $R_2$, $R_3$, $R_4$, $R_5$ und $R_6$ jeweils infrage kommen; Fluor, Chlor, Brom, $C_1$-$C_4$-Alkyl, $C_1$-$C_4$-Alkoxy.

Besonders bevorzugt sind Verbindungen der Formel (I), in welchen

- $R_1$ für Wasserstoff, Fluor, Chlor, Brom, Methyl, Ethyl, n-Propyl, i-Propyl, n-Buyl, s-Butyl, i-Butyl, t-Butyl, Phenyl oder Naphthyl steht,
- $R_2$ für Wasserstoff, Fluor, Chlor, Brom, Methyl, Ethyl, Phenyl oder Naphthyl steht,
- $R_3$ für Wasserstoff, Fluor, Chlor, Brom, Methyl oder Ethyl steht,
- $R_4$ für Wasserstoff, Fluor, Chlor, Brom, Cyano, Methyl, Ethyl, Phenyl, Methoxycarbonyl oder Ethoxycarbonyl steht,
- $R_5$ für Methyl, Ethyl, n-Propyl, i-Propyl, n-Butyl, i-Butyl, s-Butyl, t-Butyl oder Phenyl steht,

$R_6$ für Methyl, Ethyl, n-Propyl, i-Propyl, n-Butyl, i-Butyl, s-Butyl, t-Butyl oder für gegebenenfalls 1 bis 3-fach gleich oder verschieden durch Fluor, Chlor, Brom, Methyl, Methoxy, Hydroxy, Trifluormethyl, Trifluormethoxy, oder Trifluormethylthio substituiertes Phenyl steht,
X für Wasserstoff, Chlor, Brom, Methyl oder Ethyl steht, und
m für 1, 2 oder 3 steht.

Ganz besonders bevorzugt sind Verbindungen der Formel (I), in welchen
$R_1$ für Wasserstoff, Fluor, Chlor oder Methyl steht,
$R_2$ für Wasserstoff oder Methyl steht,
$R_3$ für Wasserstoff,
$R_4$ für Wasserstoff,
$R_5$ für Wasserstoff, Fluor, Chlor oder Methyl steht,
$R_6$ für Methyl, Ethyl, n-Propyl, i-Propyl, n-Butyl, i-Butyl, s-Butyl, t-Butyl oder für gegebenenfalls 1 oder 2-fach, gleich oder verschieden durch Fluor, Chlor oder Trifluormethyl substituiertes Phenyl steht, und
m für 3 steht.

Verwendet man beispielsweise 2-Trifluormethyl-2-methyl-pyrrolidin und 2,6-Dichlorbenzoesäurechlorid als Ausgangsstoffe, so läßt sich der Reaktionsablauf des erfindungsgemäßen Verfahrens durch das folgende Formelschema darstellen:

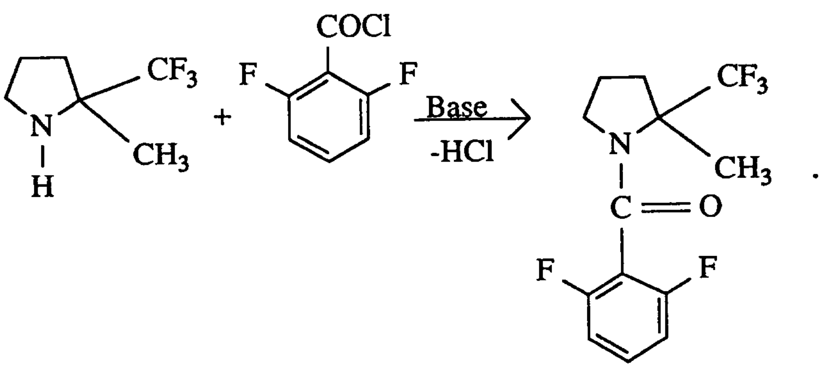


Die beim erfindungsgemäßen Verfahren zur Herstellung von Verbindungen der Formel (I) als Ausgangsstoffe zu verwendenden Pyrrolidin-Derivate sind durch die Formel (II) allgemein definiert.

In Formel (II) haben $R_1$, $R_2$, $R_3$, $R_4$, $R_5$, X und m vorzugsweise bzw. insbesondere diejenige Bedeutung, die bereits oben im Zusammenhang mit der Beschreibung der erfindungsgemäßen Verbindungen der Formel (I), vorzugsweise bzw. als insbesondere bevorzugt für $R_1$, $R_2$, $R_3$, $R_4$, $R_5$, X und m, angegeben wurden.

Man erhält die neuen Pyrrolidin-Derivate der Formel (II), indem man

a) Nitroalkylverbindungen der Formel (IV)

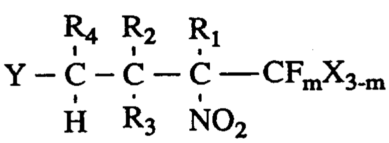 (IV),

in welcher
$R_1$, $R_2$, $R_3$, $R_4$, X und m die oben angegebene Bedeutung haben, und
Y für $-COR_5$ oder -CN steht,
worin
$R_5$ die oben angegebene Bedeutung hat,
intermediär zu den entsprechenden Aminoalkylverbindungen der Formel

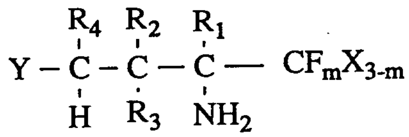 (V),

in welcher
$R_1$, $R_2$, $R_3$, $R_4$, X, m und Y die oben angegebene Bedeutung haben,
reduziert, und die Verbindungen der Formel (V), vorzugsweise ohne Zwischenisolierung, gegebenenfalls in Gegenwart eines Verdünnungsmittels wie beispielweise Methanol oder Ethanol, bei Temperaturen zwischen 25 und 150 °C, vorzugsweise zwischen 40 und 90 °C, cyclisiert. Als Reduktionsmittel können beispielsweise alle gängigen Reduktionsmittel wie beispielsweise komplexe Alkalimetallhydride, wie Lithiumaluminiumhydrid, verwendet werden.

Vorzugsweise verwendet man Wasserstoff unter einem Partialdruck von 5 bis 150 bar, vorzugsweise 40 bis 80 bar, in Gegenwart von geeigneten Katalysatoren wie beispielsweise Raney-Nickel, Palladium/Aktivkohle oder Kupferchromit.

Die Aufarbeitung kann beispielsweise durch Filtration, Extraktion des Pyrrolidin-Derivat, beispielsweise mit Ether, und anschließender Destillation erfolgen.

Es ist auch möglich, nach der Filtration das Pyrrolidin-Derivat der Formel (II) durch Zusatz von wäßriger Salzsäure als Hydrochlorid auszufällen. Aus dem Hydrochlorid kann anschließend durch Behandeln mit wäßriger Alkalihydroxidlösung wieder das Pyrrolidin-Derivat (II) freigesetzt werden, welches durch Extraktion z.B. mit Diethylether und anschließender Destillation isoliert werden kann.

Die Pyrrolidin-Derivate der Formel (II) fallen als Racemate/Diastereomerengemisch an. Eine Trennung oder Anreicherung ist nach bekannten Verfahren möglich (vgl. DE-OS 3 739 784).

Die Pyrrolidin-Derivate der Formel (II) können auch erhalten werden, wenn man
b) Pyrrolidinon-Derivate der Formel (VI)

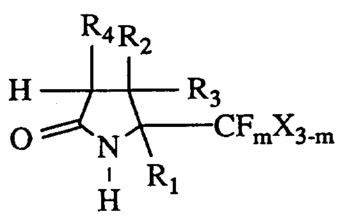 (VI),

in welcher
$R_1$, $R_2$, $R_3$, $R_4$, X und m die oben angegebene Bedeutung haben,
unter drastischen Bedingungen hydriert, beispielsweise mit Wasserstoff bei einem Druck von 150-250 bar, vorzugsweise bei 180-200 bar, in Gegenwart eines geeigneten Katalysators wie beispielsweise Raney-Nickel oder Kupferchromit, bei Temperaturen zwischen 150 und 280 °C, vorzugsweise 180 und 220 °C, gegebenenfalls in Gegenwart eines Verdünnungsmittels wie beispielsweise Methanol oder Wasser.

Die Pyrrolidinon-Derivate sind durch die Formel (VI) allgemein definiert.

In Formel (VI) haben $R_1$, $R_2$, $R_3$, $R_4$, X und m vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R_1$, $R_2$, $R_3$, $R_4$, X und m angegeben wurden.

Die neuen Pyrrolidinon-Derivate der Formel (VI) können hergestellt werden, indem man Nitroalkylverbindungen der Formel (VII)

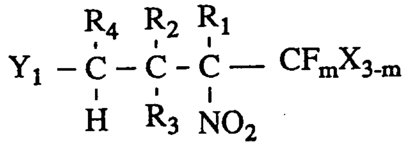 (VII),

in welcher
$R_1$, $R_2$, $R_3$, $R_4$, X und m die oben angegebene Bedeutung haben, und
$Y_1$ für $-COOR_7$ steht,
worin
$R_7$ für Wasserstoff oder $C_1$-$C_6$-Alkyl steht,
intermediär zu den entsprechenden Aminoalkylverbindungen der Formel (VIII)

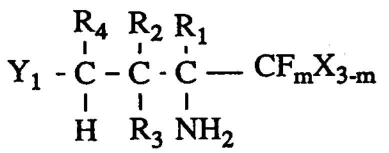
(VIII),

in welcher
$R_1$, $R_2$, $R_3$, $R_4$, X, m und $Y_1$ die oben angegebene Bedeutung haben,
reduziert, und die Verbindungen der Formel (VIII), vorzugsweise ohne Zwischenisolierung, gegebenenfalls in Gegenwart eines Verdünnungsmittels wie z.B. Alkoholen, wie Methanol, Ethern wie Diethylether, Tetrahydrofuran, Dioxan oder Wasser, bei Temperaturen zwischen 25 und 130 °C, vorzugsweise 50 und 90 °C, cyclisiert.

Als Reduktionsmittel kommen beispielsweise komplexe Alkalimetallhydride in Frage wie Lithiumaluminiumhydrid oder Wasserstoff bei einem Druck von 5-150 bar, vorzugsweise 60-90 bar, in Gegenwart geeigneter Katalysatoren wie Edelmetallkatalysatoren der 8.-Nebengruppe auf Trägermaterialien wie z.B. Bariumsulfat, Aluminiumoxid oder Kohle, weiterhin Raney-Nickel, Raney-Cobalt. Vorzugsweise verwendet man Palladium/Aktivkohle.

Die Aufarbeitung kann beispielsweise durch Filtration, Entfernung des Verdünnungsmittels und Destillation bzw. Umkristallisation erfolgen.

Die Pyrrolidinon-Derivate der Formel (VI) fallen als Racemate/Diasteromerengemischean. Eine Trennung oder Anreicherung ist nach bekannten Verfahren möglich (vgl. z.B. DE-OS 3 739 784).

Die Nitroalkylverbindungen der Formeln (IV) und (VIII) sind bekannt und/oder können analog zu bekannten Verfahren hergestellt werden (vgl. z.B. DE-OS 3 739 784; DE-OS 3 808 276).

Die beim erfindungsgemäßen Verfahren zur Herstellung von Verbindungen der Formel (I) außerdem als Ausgangsstoffe zu verwendenden Säurehalogenide sind durch die Formel (III) allgemein definiert.

In Formel (III) hat $R_6$ vorzugsweise bzw. insbesondere diejenige Bedeutung, die bereits oben im Zusammenhang mit der Beschreibung der erfindungsgemäßen Verbindungen der Formel (I), vorzugsweise bzw. als insbesondere bevorzugt für $R_6$, angegeben wurde. Z steht für Halogen, insbesondere für Chlor oder Brom.

Säurehalogenide der Formel (III) sind allgemein bekannte Verbindungen der organischen Chemie.

Als Verdünnungsmittel zur Durchführung des erfindungsgemäßen Verfahrens kommen vorzugsweise alle inerten organischen Lösungsmittel infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether, wie Diethylether, Diisopropylether, Dioxan und Tetrahydrofuran, Ketone, wie Aceton, Butanon, Methylisopropyl- und Methylisobutylketon, Nitrile, wie Acetonitril und Propionitril sowie die hochpolaren Lösungsmittel Dimethylsulfoxid und Hexamethylphosphorsäuretriamid.

Das erfindungsgemäße Verfahren wird vorzugsweise in Gegenwart eines geeigneten Säurebindemittels durchgeführt. Als solche kommen alle üblicherweise verwendbaren anorganischen und organischen Basen infrage. Vorzugsweise verwendet man Alkalimetallalkoholate wie Natrium- oder Kalium-tert-butylat, Natrium- oder Kalium-tert-amylat, Alkalimetallcarbonate wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und 1,5-Diazabicyclo(4,3,0)-non-5-en.

Die Reaktionstemperaturen können bei dem erfindungsgemäßen Verfahren in einem größeren Bereich variiert werden. Im allgemeinen arbeitet man bei Temperaturen zwischen -30 °C und +200 °C, vorzugsweise bei Temperaturen zwischen 0 °C und +110 °C.

Das erfindungsgemäße Verfahren wird im allgemeinen bei Normaldruck durchgeführt. Zur Durchführung des erfindungsgemäßen Verfahrens setzt man die zu verwendenden Ausgangsstoffe im allgemeinen in angenähert äquimolaren Mengen ein. Ein Überschuß der einen oder anderen Komponente bis etwa 10% ist

jedoch problemlos möglich.

Bei dem erfindungsgemäßen Verfahren wird die Umsetzung vorzugsweise unter Verwendung eines der oben genannten Säurebindemittel in einem der oben angegebenen Verdünnungsmitteln durchgeführt. Das Reaktionsgemisch wird eine Stunde bei der erforderlichen Temperatur gerührt. Die Aufarbeitung der Reaktionsmischung und die Isolierung der erfindungsgemäßen Reaktionsprodukte der Formel (I) erfolgt in allgemein üblicher Art und Weise.

Die erfindungsgemäßen Wirkstoffe der Formel (I) weisen eine starke Wirkung gegen Nematoden auf und können weiterhin zur Bekämpfung von unerwünschtem Pflanzenwuchs praktisch eingesetzt werden. Die Wirkstoffe sind für den Gebrauch als Pflanzenschutzmittel, insbesondere als Herbizide und Nematozide, geeignet.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden.

Dikotyle Unkräuter der Gattungen: Sinapis, Lepidium, Galium, Stellaria, Matricaria, Anthemis, Galinsoga, Chenopodium Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cyanodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfüng in einjährigen Kulturen eingesetzt werden.

Weiterhin eignen sich die erfindungsgemäßen Wirkstoffe vorzüglich zur Bekämpfung von tierischen Schädlingen, vorzugsweise von Nematoden.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp., Globodera rostochiensis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon der Cyclohexanon, stark polare Lösungsmittel, wie Dimethylformamid und Dimethylsulfoxid oder wie Wasser. Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und

fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendungen finden, wobei Fertigformulierungen oder Tankmischungen möglich sind.

Auch eine Mischung mit anderen bekannten Wirkstoffen, wie Fungtziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Herstellungsbeispiele

Beispiel 1

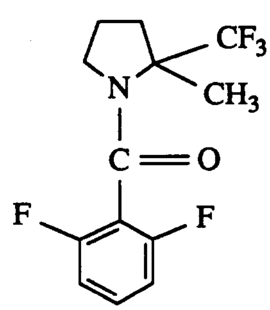

Zu einer Lösung von 7,65 g (0,05 Mol) 2-Trifluormethyl-2-methylpyrrolidin in 25 ml Tetrahydrofuran (THF) wurde eine Lösung von 8,83 g (0,05 Mol) 2,6-Difluorbenzoesäurechlorid in 10 ml Tetrahydrofuran (THF), der 5,6 g (0,055 Mol) Triethylamin zugesetzt waren, getropft. Die Reaktion ist exotherm, durch langsames Zutropfen wurde ein Ansteigen der Temperatur über +35 °C vermieden. Es wurde eine Stunde bei Raumtemperatur nachgerührt, dann auf 250 ml Eiswasser gegossen, mit verdünnter Salzsäure sauer gestellt, der erhaltene Niederschlag abgesaugt, gut mit Wasser nachgewaschen und getrocknet. Man erhält 9,96 g (68 % Ausbeute d.Th.) an N-(2,6-Difluorbenzoyl)-2-trifluormethyl-2-methyl-pyrrolidin vom Schmelzpunkt 119-120 °C.

Beispiel 2

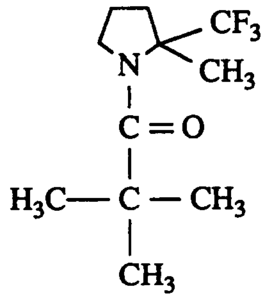

Zu einer Lösung von 7,65 g (0,05 Mol) 2-Trifluormethyl-2-methylpyrrolidin in 15 ml THF, der 5,6 g (0,055 Mol) Triethylamin zugesetzt waren, wurde eine Lösung von 6,03 g (0,05 Mol) 2,2-Dimethylpropionsäurechlorid in 25 ml THF getropft. Die Reaktion verläuft exotherm. Es wurde so langsam zugetropft, daß die Temperatur nicht über +35 °C anstieg. Es wurde eine Stunde bei Raumtemperatur nachgerührt, dann auf 200 ml Eiswasser gegossen, mit verdünnter Salzsäure auf pH 4-5 gestellt und zweimal mit je 60 ml Dichlormethan extrahiert. Die vereinigten organischen Phasen wurden über Magnesiumsulfat getrocknet, filtriert und das Lösungsmittel im Wasserstahlvakuum abgezogen. Das zurückbleibende Öl wurde über eine kurze Kieselgel-Säule (Laufmittel Toluol/Essigester 1:1) chromatografiert. Nach Entfernen des Laufmittels erhält man 9,12 g (77 % Ausbeute d.Th.) an N-Pivaloyl-2-trifluormethyl-2-methylpyrrolidin als hellgelbes Öl mit dem Brechungsindex von
$n_D^{20}$ : 1.4615 (Reinheit nach GC: 97.7%).

Beispiel 3

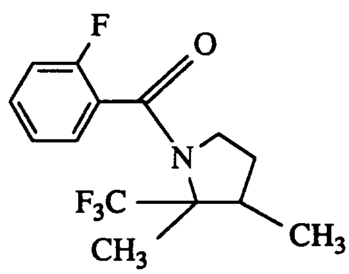

1,6 g (0,01 Mol) 2-Fluorbenzoylchlorid wurden in 100 ml absolutem Tetrahydrofuran gelöst. Bei 0 °C wurde eine Lösung von 1,4 ml (0,01 Mol) Triethylamin und 1,67 g (0,01 Mol) 2,3-Dimethyl-2-trifluormethyl-pyrrolidin, gelöst in 10 ml absolutem Tetrahydrofüran, langsam zugetropft Nach beendeter Zugabe ließ man den Ansatz auf Raumtemperatur kommen und rührte eine Stunde nach. Zur Aufarbeitung wurde mit 50 ml Methylenchlorid versetzt und dann nacheinander mit 2 N Salzsäure, gesättigter $NaHCO_3$-Lösung und gesättigter NaCl-Lösung gewaschen. Zur Reinigung des Rohprodukts wurde chromatographiert (Laufmittelgemisch: Essigester/Cyclohexan 1:2).

Man erhält: 2,5 g (86 %) an N-(2-Fluorbenzoyl)-2,3-dimethyl-2-trifluormethyl-pyrrolidin mit den Daten:
$^1$H-NMR($CDCl_3$, 200 MHz): 1,115 d, J = 7Hz (3H);
1,35-1,95 m (2H); 2,60 m (1H); 1,70 s (3H);
3,35 m (2H); 7,04-7,38 m (4H).

Analog zu den Herstellungsbeispielen und gemäß den allgemeinen Ausgaben zu dem erfindungsgemäßen Verfahren können die in Tabelle 1 aufgeführten Verbindungen der allgemeinen Formel (I) hergestellt werden

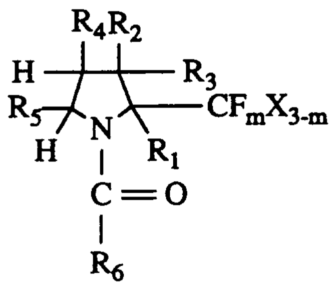

(I).

<u>Tabelle 1</u>

| Bsp. Nr. | $R_1$ | $R_2$ | $R_3$ | $R_4$ | $R_5$ | $R_6$ | $-CF_mX_{3-m}$ | Physical. Daten |
|---|---|---|---|---|---|---|---|---|
| 4 | $CH_3$ | $CH_3$ | H | H | H | 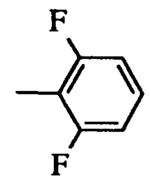 | $CF_3$ | Smp. 66-67°C |
| 5 | $CH_3$ | H | H | H | $CH_3$ | 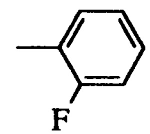 | $CF_3$ | Smp. 94-96°C |
| 6 | $CH_3$ | H | H | H | $CH_3$ | 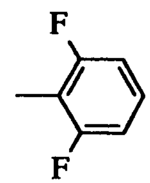 | $CF_3$ | Smp. 103-104°C |
| 7 | $CH_3$ | H | H | H | H | 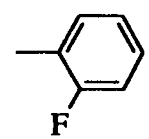 | $CF_3$ | Öl, IR:2980; 1660; 1455 $cm^{-1}$ |
| 8 | H | H | H | H | $CH_3$ | 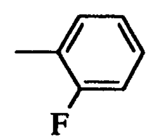 | $CF_3$ | Öl, IR: 2950; 1655; 1380 $cm^{-1}$ |

Tabelle 1

| Bsp. Nr. | $R_1$ | $R_2$ | $R_3$ | $R_4$ | $R_5$ | $R_6$ | $-CF_mX_{3-m}$ | Physical. Daten |
|---|---|---|---|---|---|---|---|---|
| 9 | H | H | H | H | $CH_3$ | 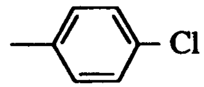 | $CF_3$ | Smp. 72-74°C |
| 10 | H | H | H | H | H | 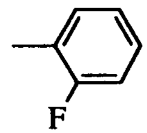 | $CF_3$ | Öl, IR: 2950; 1660; 1390 $cm^{-1}$ |
| 11 | $CH_3$ | H | H | H | H | 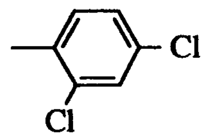 | $CF_3$ | Smp. 92-93°C |
| 12 | $CH_3$ | H | H | H | $CH_3$ | 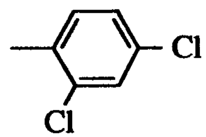 | $CF_3$ | Smp. 92-94°C |
| 13 | $CH_3$ | $CH_3$ | H | H | H | 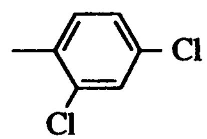 | $CF_3$ | Smp. 58-60°C |
| 14 | H | H | H | H | $CH_3$ | 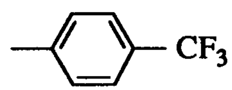 | $CF_3$ | Öl, IR: 2970; 1660; 1385 $cm^{-1}$ |

Herstellung der Ausgangsverbindungen

a) der Formel (II)

Beispiel 15

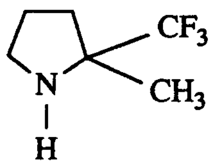

Variante A

100 g (0,5 Mol) 4-(Trifluormethyl)-4-nitropentan-1-al wurden in 300 ml Methanol an 10 g Raney-Nickel bei 80 °C 8 Stunden lang in einem 0,7 l fassenden V4A-Rührautoklaven hydriert. Der Wasserstoffpartialdruck betrug 80 bar; es wurden 90 % der theoretischen Menge Wasserstoff aufgenommen. Zur Aufarbeitung wurde der Katalysator abfiltriert und mit wenig Methanol nachgewaschen. Die methanolische Lösung wurde mit 100 ml konzentrierter Salzsäure sauer gestellt und bis zur Trockne unter vermindertem Druck eingeengt. Es resultierten 59 g (0,34 Mol) an 2-Trifluormethyl-2-methyl-pyrrolidin-Hydrochlorid (67 % der Theorie). Aus dem Hydrochlorid wurde durch Zugabe einer äquimolaren Menge Natriumhydroxid-Lösung, Extraktion mit Diethylether, Trocknung über Natriumsulfat und anschließender destillativer Entfernung des Lösungsmittels über eine 30 cm lange Vigreux-Kolonne das 2-Trifluormethyl-2-methyl-pyrrolidin erhalten ($Kp_{1013}$: 115-118 °C).

Beispiel 16

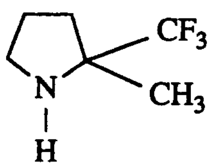

Variante B

98 g (0,5 Mol) 4-(Trifluormethyl)-4-nitropentancarbonsäurenitril wurden in 300 ml Methanol an 10 g Raney-Nickel bei 100 °C 8 Stunden in einem 0,7 l fassenden V4A-Rührautoklaven hydriert. Der Wasserstoffpartialdruck betrug 70-80 bar, es wurden 85 % der theoretischen Menge Wasserstoff aufgenommen. Zur Aufarbeitung wurde der Katalysator abfiltriert und mit wenig Methanol gewaschen. Im weiteren wurde wie in Beispiel 15 verfahren. Es wurden 57 g (0,3 Mol) an 2-Trifluormethyl-2-methyl-pyrrolidin-Hydrochlorid erhalten (60 % der Theorie).

Beispiel 17

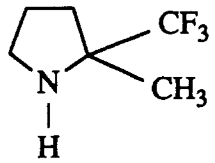

Variante C

41,5 g (0.25 Mol) 5-Trifluormethyl-5-methyl-pyrrolidin-2-on wurden in 100 ml Methanol an 5 g Kupferchromit ($CuCr_2O_4$) 16 Stunden bei 180-190 °C in einem 0.3 l V4A-Rührautoklaven hydriert. Der Wasserstoffpartialdruck betrug 220-240 bar; es wurden 80 % der theoretischen Menge Wasserstoff aufgenommen. Die Aufarbeitung erfolgte analog Beispiel 15.

Es wurden 25 g (0.13 Mol) 2-Trifluormethyl-2-methyl-pyrrolidin-Hydrochloriderhalten (53 % der Theorie).

Analog zu den Beispielen 15, 16 und 17 und gemäß den allgemeinen Angaben können die in Tabelle 2 aufgeführten Verbindungen der allgemeinen Formel (II) hergestellt werden

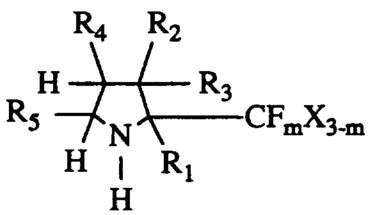

(II).

Tabelle 2

| Bsp. Nr. | Variante | $R_1$ | $R_2$ | $R_3$ | $R_4$ | $R_5$ | $-CF_mX_{3-m}$ | Physical. Daten |
|---|---|---|---|---|---|---|---|---|
| 18 | A | H | H | H | H | $CH_3$ | $CF_3$ | $Kp_{1013}$: 118-120°C |
| 19 | A | $CH_3$ | H | H | H | $CH_3$ | $CF_3$ | $Kp_{1013}$: 125-126°C |
| 20 | A | $CH_3$ | $CH_3$ | H | H | H | $CF_3$ | $Kp_{1013}$: 121-124°C |
| 21 | A,C | H | H | H | H | H | $CF_3$ | *) |

*) siehe Isv. Akad. Nauk SSSR, Ser. Khim, 1422 (1988)

b) der Formel (VI)

Beispiel 22

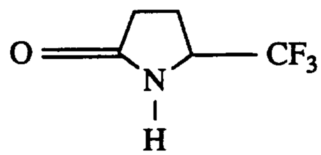

108 g (0,50 Mol) 5,5,5-Trifluor-4-nitropentancarbonsäuremethylester wurden in 300 ml Methanol an 10 g 5 %igem Palladium auf Kohle bei 60 °C 6 Stunden lang in einem 0,7 l fassenden V4A-Rührautoklaven hydriert Der Wasserstoffpartialdruck betrug 60 bar, es wurden 95 % der theoretischen Menge Wasserstoff aufgenommen. Zur Aufarbeitung wurde der Katalysator abfiltriert und mit wenig Methanol nachgewaschen. Das Lösungsmittel wurde im Wasserstrahlvakuum zuerst bei 40 °C, dann bei 80 °C destillativ entfernt. Dabei begann sich der Rückstand zu verfestigen. Zur weiteren Reinigung wurde im Wasserstrahlvakuum destilliert.

Es wurden 57 g (0,37 Mol) 5-Trifluormethyl-pyrrolidin-2-on vom Kochpunkt $Kp_{14}$: 122-124 °C erhalten (74,5 % der Theorie).

Beispiel 23

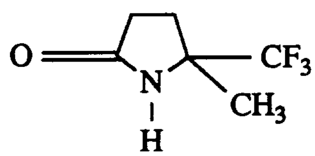

Analog zu Beispiel 22 wurden 115 g (0,5 Mol) 4-Trifluormethyl-4-nitropentancarbonsäuremethylester umgesetzt und aufgearbeitet.

Nach Umkristallisation aus Ligroin wurden 72 g (0,43 Mol) 5-Trifluormethyl-5-methyl-pyrrolidin-2-on vom Schmelzpunkt 111-112 °C erhalten (86 % der Theorie).

Anwendungsbeispiele

Beispiel A-1

Ein Gew.-Teil der nach Beispiel 9 erhaltenen Verbindung wurde mit 4 Gew.-Teilen Aceton vermischt, 1 Gew.-Teil Alkylarylpolyglykolether als Emulgator hinzugegeben und die Mischung mit Wasser verdünnt. So wurde eine Präparation einer aktiven Verbindung erhalten.

Diese Präparation wurde innig mit Erde vermischt, die mit Globodera rostochiensis (= Testnematoden) stark verseucht war. Die Konzentration der aktiven Verbindung in der Präparation ist praktisch von keiner Bedeutung, nur die Menge der aktiven Verbindung pro Volumeneinheit der Erde ist entscheidend. Diese betrug 20 ppm (= mg/l). Die so behandelte Erde wurde in Töpfe gefüllt, Kartoffeln wurden eingepflanzt und die Töpfe in einem Gewächshaus bei 20 °C gehalten.

Nach 6 Wochen wurden die Kartoffelnwurzeln auf Zysten untersucht. Es wurde gefunden, daß die Präparation der aktiven Verbindung 95 % des Befalls unterdrückt hatte im Vergleich zu Kontrollpflanzen in unbehandelter, aber in gleicher Weise verseuchter Erde.

Beispiel A-2

Ein Gew.-Teil der nach Beispiel 14 erhaltenen Verbindung wurde mit 4 Gew.-Teilen Aceton vermischt, 1 Gew.-Teil Alkylarylpolyglykolether als Emulgator hinzugegeben und die Mischung mit Wasser verdünnt. So

wurde eine Präparation einer aktiven Verbindung erhalten.

Diese Präparation wurde innig mit Erde vermischt, die mit Meloidogyne incognita (= Testnematoden) stark verseucht war. Die Konzentration der aktiven Verbindung in der Präparation ist praktisch von keiner Bedeutung, nur die Menge der aktiven Verbindung pro Volumeneinheit der Erde ist entscheidend. Diese betrug 20 ppm (=mg/l). Die so behandelte Erde wurde in Töpfe gefüllt, Salat wurde eingesät und die Töpfe in einem Gewächshaus bei 25 °C gehalten.

Nach 4 Wochen wurden die Salatwurzeln auf Zysten untersucht Es wurde gefunden, daß die Präparation der aktiven Verbindung 95 % des Befalls unterdrückt hatte im Vergleich zu Kontrollpflanzen in unbehandelter, aber in gleicher Weise verseuchter Erde.

## Patentansprüche

**1.** N-Acylpyrrolidin-Derivate der Formel

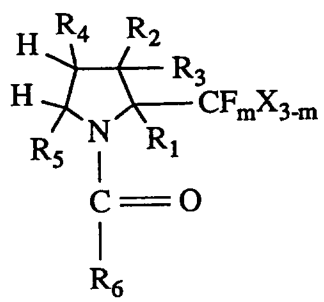

(I),

in welcher

| | |
|---|---|
| $R_1$, $R_2$ und $R_3$ | unabhängig voneinander für Wasserstoff, Halogen, Alkyl oder Aryl stehen, |
| $R_4$ | für Wasserstoff, Halogen, Cyano, Alkyl, Aryl oder 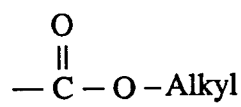 steht, |
| $R_5$ | für Wasserstoff, Alkyl oder Aryl steht, |
| $R_6$ | für Alkyl oder Aryl steht, |
| X | für Wasserstoff, Halogen oder Alkyl steht, und |
| m | für 1, 2 oder 3 steht. |

**2.** Verbindungen gemäß Anspruch 1, in welchen

| | |
|---|---|
| $R_1$, $R_2$ und $R_3$ | unabhängig voneinander für Wasserstoff, Fluor, Chlor, Brom, gegebenenfalls substituiertes $C_1$-$C_6$-Alkyl oder gegebenenfalls substituiertes $C_6$-$C_{10}$-Aryl stehen, |
| $R_4$ | für Wasserstoff, Fluor, Chlor, Brom, Cyano, gegebenenfalls substituiertes $C_1$-$C_6$-Alkyl, gegebenenfalls substituiertes $C_6$-$C_{10}$-Aryl oder 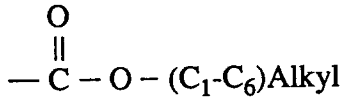 steht, |
| $R_5$ | für Wasserstoff, gegebenenfalls substituiertes $C_1$-$C_6$-Alkyl oder gegebenenfalls substituiertes $C_6$-$C_{10}$-Aryl steht, |
| $R_6$ | für gegebenenfalls substituiertes $C_1$-$C_6$-Alkyl oder gegebenenfalls substituiertes $C_6$-$C_{10}$-Aryl steht, |
| X | für Wasserstoff, Fluor, Chlor, Brom oder gegebenenfalls substituiertes $C_1$-$C_4$-Alkyl |

steht, und

m für 1, 2 oder 3 steht,

wobei als Alkylsubstituenten in den Definitionen von $R_1$, $R_2$, $R_3$, $R_4$, $R_5$, $R_6$ und X jeweils infrage kommen: Fluor, Chlor, Brom, Hydroxy, Methoxy oder Ethoxy, und als Arylsubstituenten in den Definitionen von $R_1$, $R_2$, $R_3$, $R_4$, $R_5$ und $R_6$ jeweils infrage kommen: Fluor, Chlor, Brom, $C_1$-$C_4$-Alkyl, $C_1$-$C_4$-Alkoxy.

**3.** Verbindungen des Anspruchs 1, in welchen

$R_1$ für Wasserstoff, Fluor, Chlor, Brom, Methyl, Ethyl, n-Propyl, i-Propyl, n-Butyl, s-Butyl, i-Butyl, t-Butyl, Phenyl oder Naphthyl steht,

$R_2$ für Wasserstoff, Fluor, Chlor, Brom, Methyl, Ethyl, Phenyl oder Naphthyl steht,

$R_3$ für Wasserstoff, Fluor, Chlor, Brom, Methyl oder Ethyl steht,

$R_4$ für Wasserstoff, Fluor, Chlor, Brom, Cyano, Methyl, Ethyl, Phenyl, Methoxycarbonyl oder Ethoxycarbonyl steht,

$R_5$ für Methyl, Ethyl, n-Propyl, i-Propyl, n-Butyl, i-Butyl, s-Butyl, t-Butyl oder Phenyl steht,

$R_6$ für Methyl, Ethyl, n-Propyl, i-Propyl, n-Butyl, i-Butyl, s-Butyl, t-Butyl oder für gegebenenfalls 1 bis 3-fach gleich oder verschieden durch Fluor, Chlor, Brom, Methyl, Methoxy, Hydroxy, Trifluormethyl, Trifluormethoxy oder Trifluormethylthio substituiertes Phenyl steht,

X für Wasserstoff, Chlor, Brom, Methyl oder Ethyl steht, und

m für 1, 2 oder 3 steht.

**4.** Verfahren zur Herstellung der neuen N-Acylpyrrolidin-Derivate des Anspruchs 1, dadurch gekennzeichnet, daß Pyrrolidin-Derivate der Formel

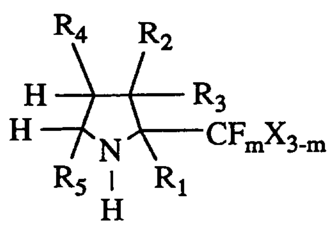

(II),

in welcher

$R_1$, $R_2$, $R_3$, $R_3$, $R_4$, $R_5$, X und m die in Anspruch 1 angegebene Bedeutung haben,

mit Säurehalogeniden der Formel

$$R_6 - \overset{O}{\overset{\|}{C}} - Z \quad \text{(III)},$$

in welcher

$R_6$ die in Anspruch 1 angegebene Bedeutung hat, und

Z für Halogen, insbesondere für Brom oder Chlor, steht,

gegebenenfalls in Gegenwart eines Verdünnungsmittels und gegebenenfalls in Gegenwart eines Säurebindemittels umgesetzt werden.

**5.** Verwendung der N-Acylpyrrolidin-Derivate des Anspruchs 1 als Herbizide und Nematozide.

## Claims

**1.** N-Acylpyrrolidine derivatives of the formula

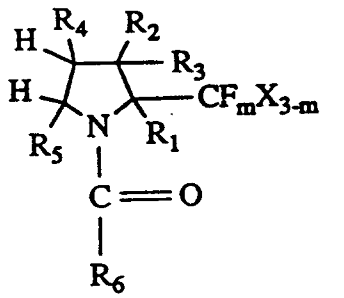

(I)

in which

| | |
|---|---|
| $R_1$, $R_2$ and $R_3$ | independently of one another represent hydrogen, halogen, alkyl or aryl, |
| $R_4$ | represents hydrogen, halogen, cyano, alkyl, aryl or |

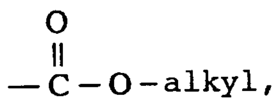

| | |
|---|---|
| $R_5$ | represents hydrogen, alkyl or aryl, |
| $R_6$ | represents alkyl or aryl, |
| X | represents hydrogen, halogen or alkyl, and |
| m | represents 1, 2 or 3. |

**2.** Compounds according to Claim 1, in which

| | |
|---|---|
| $R_1$, $R_2$ and $R_3$ | independently of one another represent hydrogen, fluorine, chlorine, bromine, optionally substituted $C_1$-$C_6$-alkyl or optionally substituted $C_6$-$C_{10}$-aryl, |
| $R_4$ | represents hydrogen, fluorine, chlorine, bromine, cyano, optionally substituted $C_1$-$C_6$-alkyl, optionally substituted $C_6$-$C_{10}$-aryl or |

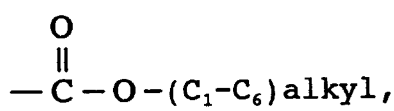

| | |
|---|---|
| $R_5$ | represents hydrogen, optionally substituted $C_1$-$C_6$-alkyl or optionally substituted $C_6$-$C_{10}$-aryl, |
| $R_6$ | represents optionally substituted $C_1$-$C_6$-alkyl or optionally substituted $C_6$-$C_{10}$-aryl, |
| X | represents hydrogen, fluorine, chlorine, bromine or optionally substituted $C_1$-$C_4$-alkyl, and |
| m | represents 1, 2 or 3, |

where the following are in each case suitable as alkyl substituents in the definitions of $R_1$, $R_2$, $R_3$, $R_4$, $R_5$, $R_6$ and X: fluorine, chlorine, bromine, hydroxyl, methoxy or ethoxy, and the following are in each case suitable as aryl substituents in the definitions of $R_1$, $R_2$, $R_3$, $R_4$, $R_5$ and $R_6$: fluorine, chlorine, bromine, $C_1$-$C_4$-alkyl or $C_1$-$C_4$-alkoxy.

**3.** Compounds of Claim 1, in which

| | |
|---|---|
| $R_1$ | represents hydrogen, fluorine, chlorine, bromine, methyl, ethyl, n-propyl, i-propyl, n-butyl, s-butyl, i-butyl, t-butyl, phenyl or naphthyl, |
| $R_2$ | represents hydrogen, fluorine, chlorine, bromine, methyl, ethyl, phenyl or naphthyl, |
| $R_3$ | represents hydrogen, fluorine, chlorine, bromine, methyl or ethyl, |
| $R_4$ | represents hydrogen, fluorine, chlorine, bromine, cyano, methyl, ethyl, phenyl, methoxycarbonyl or ethoxycarbonyl, |

| | |
|---|---|
| $R_5$ | represents methyl, ethyl, n-propyl, i-propyl, n-butyl, i-butyl, s-butyl, t-butyl or phenyl, |
| $R_6$ | represents methyl, ethyl, n-propyl, i-propyl, n-butyl, i-butyl, s-butyl or t-butyl, or represents phenyl which is optionally mono-substituted to trisubstituted by identical or different substituents from the series comprising fluorine, chlorine, bromine, methyl, methoxy, hydroxyl, trifluoromethyl, trifluoromethoxy and trifluoromethylthio, |
| X | represents hydrogen, chlorine, bromine, methyl or ethyl, and |
| m | represents 1, 2 or 3. |

**4.** Process for the preparation of the novel N-acylpyrrolidine derivatives of Claim 1, characterised in that pyrrolidine derivatives of the formula

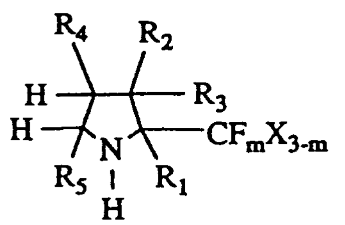

(II)

in which
$R_1$, $R_2$, $R_3$, $R_4$, $R_5$, $R_6$, X and m have the meaning given in Claim 1,
are reacted with acid halides of the formula

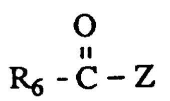

(III),

(III),

in which

| | |
|---|---|
| $R_6$ | has the meaning given in Claim 1 and |
| Z | represents halogen, in particular bromine or chlorine, |

if appropriate in the presence of a diluent and if appropriate in the presence of an acid-binding agent.

**5.** Use of the N-acylpyrrolidine derivatives of Claim 1 as herbicides and nematicides.

**Revendications**

**1.** Dérivés de N-acylpyrrolidines de formule

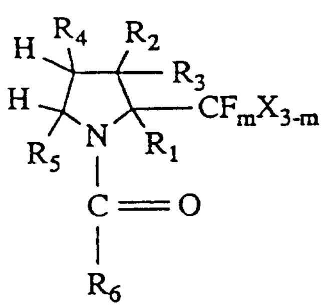

(I),

dans laquelle

| | |
|---|---|
| $R_1$, $R_2$ et $R_3$ | représentent indépendamment l'un de l'autre l'hydrogène, un halogène ou un groupe alkyle ou aryle, |
| $R_4$ | représente l'hydrogène, un halogène ou un groupe cyano, alkyle, aryle ou |

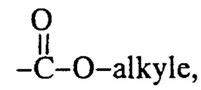

R5 représente l'hydrogène ou un groupe alkyle ou aryle,
R6 représente un groupe alkyle ou aryle,
X représente l'hydrogène, un halogène ou un groupe alkyle et
m représente 1, 2 ou 3.

**2.** Composés selon la revendication 1, dans lesquels

- $R_1$, $R_2$ et $R_3$ représentent indépendamment l'un de l'autre l'hydrogène, le fluor, le chlore, le brome, un groupe alkyle en $C_1$-$C_6$ éventuellement substitué ou un groupe aryle en $C_6$-$C_{10}$ éventuellement substitué,
- $R_4$ représente l'hydrogène, le fluor, le chlore, le brome, un groupe cyano, un groupe alkyle en $C_1$-$C_6$ éventuellement substitué, un groupe aryle en $C_6$-$C_{10}$ éventuellement substitué ou un groupe

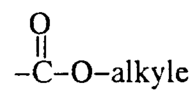

en $C_1$-$C_6$,
- $R_5$ représente l'hydrogène, un groupe alkyle en $C_1$-$C_6$ éventuellement substitué ou un groupe aryle en $C_6$-$C_{10}$ éventuellement substitué,
- $R_6$ représente un groupe alkyle en $C_1$-$C_6$ éventuellement substitué ou un groupe aryle en $C_6$-$C_{10}$ éventuellement substitué,
- X représente l'hydrogène, le fluor, le chlore, le brome ou un groupe alkyle en $C_1$-$C_4$ éventuellement substitué et
- m représente 1, 2 ou 3,

dans lesquels les substituants des groupes alkyles entrant chaque fois en considération dans les définitions de $R_1$, $R_2$, $R_3$, $R_4$, $R_5$, $R_6$ et X sont les suivants : fluor, chlore, brome, hydroxy, méthoxy ou éthoxy et les substituants des groupes aryles entrant chaque fois en considération dans les définitions de $R_1$, $R_2$, $R_3$, $R_4$, $R_5$ et $R_6$ sont les suivants : fluor, chlore, brome, alkyle en $C_1$-$C_4$, alcoxy en $C_1$-$C_4$.

**3.** Composés selon la revendication 1, dans lesquels

- $R_1$ représente l'hydrogène, le fluor, le chlore, le brome ou un groupe méthyle, éthyle, n-propyle, i-propyle, n-butyle, s-butyle, i-butyle, t-butyle, phényle ou naphtyle,
- $R_2$ représente l'hydrogène, le fluor, le chlore, le brome ou un groupe méthyle, éthyle, phényle ou naphtyle,
- $R_3$ représente l'hydrogène, le fluor, le chlore, le brome ou un groupe méthyle ou éthyle,
- $R_4$ représente l'hydrogène, le fluor, le chlore ou le brome ou un groupe cyano, méthyle, éthyle, phényle, méthoxycarbonyle ou éthoxycarbonyle,
- $R_5$ représente un groupe méthyle, éthyle, n-propyle, i-propyle, n-butyle, i-butyle, s-butyle, t-butyle ou phényle,
- $R_6$ représente un groupe méthyle, éthyle, n-propyle, i-propyle, n-butyle, i-butyle, s-butyle, t-butyle ou un groupe phényle éventuellement substitué par 1 à 3 substituants identiques ou différents choisis parmi le fluor, le chlore ou le brome et les groupes méthyle, méthoxy, hydroxy, trifluorométhyle, trifluorométhoxy et trifluorométhylthio,
- X représente l'hydrogène, le chlore, le brome ou un groupe méthyle ou éthyle et
- m représente 1, 2 ou 3.

**4.** Procédé pour la fabrication des nouveaux dérivés de N-acylpyrrolidines selon la revendication 1, caractérisé en ce que l'on fait réagir des dérivés de pyrrolidine de formule

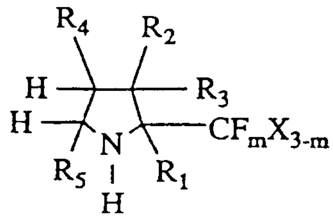 (II),

dans laquelle
$R_1$, $R_2$, $R_3$, $R_4$, $R_5$, X et m ont la signification indiquée à la revendication 1 avec des halogénures d'acides de formule

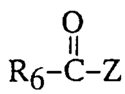 (III)

dans laquelle

- $R_6$ a la signification indiquée à la revendication 1 et
- Z représente un halogène, en particulier le brome ou le chlore,

éventuellement en présence d'un agent diluant et éventuellement en présence d'un accepteur d'acide.

**5.** Utilisation des dérivés de N-acylpyrrolidines selon la revendication 1 comme herbicides et nématocides.